# EUROPEAN PATENT SPECIFICATION

(11) **EP 0 256 871 B1**
(45) Date of publication and mention of the grant of the patent: **21.07.1993**
(21) Application number: 87307210.2
(22) Date of filing: 14.08.1987
(51) Int. Cl.: A61F 13/15

(54) **Process for making formed flexible plastic foam products**
Verfahren zum Herstellen geformter flexibler Artikel aus Schaumkunststoff
Procédé de fabrication de produits flexibles plastiques en mousse

(30) Priority: 15.08.1986 US 897223
(43) Date of publication of application: 24.02.1988
(73) Proprietor: McNEIL-PPC, INC., New Brunswick, New Jersey 08933 (US)
(72) Inventor: Menard, Michael J., Doylestown, PA 18901 (US); Marbach, Philip J., Doylestown, PA 18901 (US); Helmstetter, Thomas J., Sr., Piscataway, NJ 08854 (US); Spano, John D., Bordentown, Twsp.NJ 08505 (US)
(74) Representative: Jones, Alan John

(56) References cited:
- EP-A- 0 139 484
- US-A- 4 079 739
- US-A- 4 372 098
- US-A- 4 554 191
- US-A- 4 728 381

## Description

This invention relates to a process for making formed flexible plastic foam products. It relates particularly to a process for making such products from ethylene-containing foam shells each containing a fibrous web insert and having a porous facing sheet heat sealed onto the perimeter of each shell.

EP-A-0 139 484 describes a disposable sanitary napkin comprising a flexible boat-shaped foam plastic shell containing a superstructure, which may be a corrugated fibrous web containing a superabsorbent. The superstructure is slightly compressible and is capable of maintaining a void volume of liquid. The superstructure may be sealed in the shell by a covering sheet. A process for producing the napkin is also disclosed.

US-A-4 372 098 discloses a method of making an applicator package. The method comprises: feeding a continuous web of material, such as one comprising an aluminum foil having an inner coating of a thermoplastic material; forming successively a plurality of recesses along the web of material; depositing a fabric applicator pad into each said recess; securing said fabric applicator to the inside portion of said recess; dispensing a predetermined quantity of product to be applied by said fabric applicator to said applicator; feeding another continuous web of material so as to be continuously aligned in a coextensive manner with said first web of material; sealing the peripheral marginal edges of each applicator package so as to form a thermoplastic hermetic closure about all sides of said recess, leaving at least one corner portion of said webs of material unsecured together; and cutting off each said applicator package from said webs of material.

US-A-4 079 739 discloses a die-cut, contoured catamenial napkin of multi-layered construction. The napkin comprises joined-together layers of sheet material assembled into a sandwich which combines a thin, soft, flexible peripheral portion for comfort with a thick central portion for increased fluid-holding capacity. The uppermost layer is a thin batt of intermingled fibers provided with unbonded low density cushion areas separated by spaced bonded areas comprised of compressed fluid-distributing channels of higher density extending generally longitudinally of the batt and intersecting the ends and edges. The central portion of the uppermost layer is upwardly deformed to provide a pad-receiving cavity in which a main fluid-holding element consisting of a relatively thick pad of absorbent material is positioned in intimate physical contact with the uppermost layer while the horizontal face portions of the side and end walls of the central pad are free of contact with neighbouring components. The outer peripheral portion of the uppermost layer, and in the preferred embodiment, the lower surface of the fluid-holding element, is adhesively secured to a fluid-impervious bottom layer by a thin discontinuous film of adhesive. Pressure-sensitive adhesive may be used on the bottom of the paid for releasable attachment to a supporting undergarment.

Conventional slow rate thermal forming of plastic foam sheet materials is generally known, such as used for making containers for packaging articles including foodstuffs. However, prior attempts to increase production rates for such products formed from flexible foam sheet materials, particularly for forming flexible ethylene-containing materials into shell shapes containing absorbent inserts at high rates, have been generally unsuccessful because of the low heat conductivity and easily extrudable characteristic of the flexible foam materials. Such problems for producing flexible foam absorbent products have now been advantageously overcome by the present invention, which provides a method for producing absorbent products using flexible foam materials at substantially increased production rates of 10 to 30 cycles per minute.

According to the present invention, there is provided a process for making a formed flexible plastic foam product containing a corrugated fibrous web insert piece, the process comprising a process for making a formed flexible plastic foam product containing a corrugated fibrous web insert piece, comprising:
(a) providing a flexible foam sheet material and rapidly heating the foam material to 130-250°F (54-121°C) temperature;
(b) die forming the heated foam sheet to provide at least one formed shell integrally attached to the foam sheet, said shell having a depth at least about 10 times the sheet original thickness, said die forming step being performed by effecting relative movement between cooperating first and second die units, said first die unit having a cavity shaped to the configuration of said shell and said second die unit having a shaped plug adapted to mate within said cavity and to draw a portion of said heated foam sheet into said cavity in response to said relative movement;
(c) providing corrugated fibrous web material and die cutting at least one web piece from the web;
(d) placing each said cut out web piece into each said formed shell;
(e) applying a thin porous facing sheet over each said web piece in the formed shell, selectively transversely slitting the porous facing sheet, and thereafter heat sealing the facing sheet onto the perimeter of each said formed shell; and
(f) cutting the formed shell and attached facing sheet at the perimeter of the shell, and thereafter removing the cut out shells from the foam sheet to provide a plurality of the formed plastic foam products.

In the preferred process, a flexible ethylene-containing foam sheet is first rapidly heated from ambient to 54.4 to 121.1°C (130 to 250°F) temperature and then die formed to provide multiple parallel shells integrally attached to the foam sheet. The shells have a depth at least about 10 times the foam sheet original thickness and preferably 12 to 20 times the sheet thickness. Also, a thick fibrous web material which preferably contains an absorbent material, is die cut to provide a plurality of web pieces, which are each placed into each of the formed shells. Next, a thin porous facing sheet is placed over the web piece in each formed shell and sealed onto the shell at its perimeter, preferably by a heat sealing step. Then, the formed shells with the attached facing sheet are die cut from the foam sheet around the perimeter of each formed shell, after which the resulting product is punched out and removed from the remaining foam and facing sheet materials, and the scrap sheets are then withdrawn for disposal. The resulting individual product pieces are then removed such as by a belt conveyor for use.

The foam sheet is preferably an ethylene-containing polymer foam material identified as Volara Type A, which is a crosslinked polyethylene foam manufactured and sold by Voltek, Inc., Lawrence, Massachusetts. The expression "ethylene-containing polymer foam" used herein includes polyethylene homopolymer and ethylene-containing copolymers, preferably containing a major proportion, by weight, of ethylene. It is preferred that the polymer present be crosslinked. Preferred comonomers, for preparing the polymers, including vinyl acetate, acrylic and methylacrylic acids and esters, such as ethyl acrylate. Blends of such polymers can be used. The foam sheet material has thickness of 1.27 to 6.35 mm (0.050 to 0.250 inch). The fibrous web material preferably has a longitudinally corrugated shape and preferably contains an absorbent material such as a polymerized acrylate powder, e.g. potassium acrylate having particle size of 50 to 1000 µm. The facing sheet is a liquid permeable generally hydrophobic film or fabric.

Typical facings include polyester nonwovens, polypropylene nonwovens, perforated films such as polyethylene film. The facing sheet is a thermally bondable sheet, preferably bondable in a temperature range compatible with the ethylene-containing foam shell.

The foam sheet is rapidly radiantly heated from ambient to 54.4 to 121.1°C (130-250°F) temperature to soften the foam material before die forming the shells while rapidly cooling the formed shells during the die forming step. An absorbent powder material is preferably deposited within the fibrous web sheet,usually within corrugations formed in the web, before die cutting the web to produce the insert pieces. The corrugated fibrous web containing the powder is inverted after die cutting the web pieces from the web sheet, so that the absorbent powder is provided on the lower or inner side of the web pieces when inserted into the formed shells.

The fibrous web is preferably moved forward continuously for a powder depositing step, and the powder-filled web is moved forward intermittently for the die cutting and subsequent production steps. The web insert pieces are crimped longitudinally during picking and placing the pieces in the formed shells. The process usually includes applying a tape strip to the convex side of the formed foam shells before cutting the shells from the foam sheet.

To provide a smooth fitting facing sheet onto the formed shells containing the web inserts, the facing sheet is selectively slit transversely at selected locations between the shells before heat sealing the facing sheet to the perimeter of the formed shells. The thin facing sheet is rapidly heated to 65.6 to 176.7°C (150 to 350° F) and heat sealed onto the formed shell upper surface. The formed shells with heat bonded facing sheet are then die cut from the foam sheet around the perimeter of the formed shell except for at least one retaining link at each end of each shell, after which the die cut foam shells are punched out from the retaining foam sheet. Preferably, the foam sheet material is made sufficiently wide to accommodate two formed shells oriented in an end-to-end pattern and includes 4 to 5 adjacent shells formed by each die stroke. The fibrous web sheet is also made sufficiently wide to accommodate at least two and preferably 2 to 4 cut out pieces oriented in end-to-end arrangement and including 4 to 5 adjacent pieces per indexed movement of the web and die stroke.

For this process the products are advantageously heat formed, assembled and heat sealed together at a rate of 10 to 30 forming and cutting cycles per minute.
The invention will be further described by reference to the following drawings, in which:
Fig. 1 shows a schematic elevation view of the overall process steps for producing flexible formed plastic foam products in accordance with the invention;
Fig. 2 shows a perspective view of the foam heating and shell forming steps;
Fig. 3 shows a perspective view of providing a powder material in the web according to a preferred embodiment of the invention.
Fig. 4 shows a perspective view of the web piece punch out and lateral transfer steps for placing the web pieces into the formed shells.
Fig. 5 shows a perspective view of slitting and heat sealing a facing sheet onto the formed shells each filled with a web piece;
Fig. 6 shows a perspective view of the shell cutting and punch out steps; and

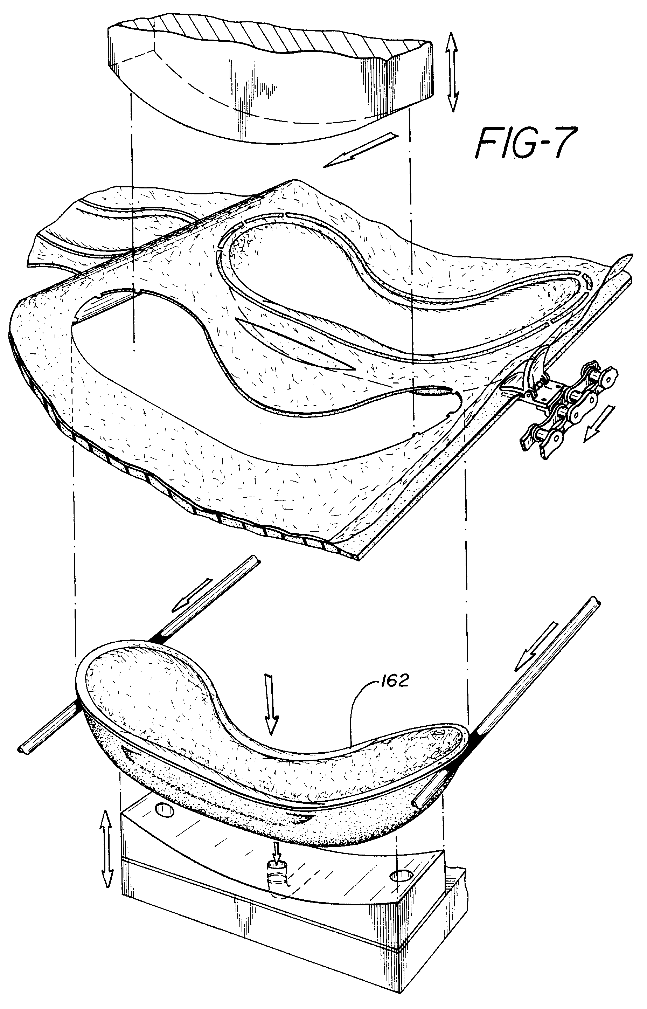

Fig. 7 shows a preferred formed shell product made according to the invention.

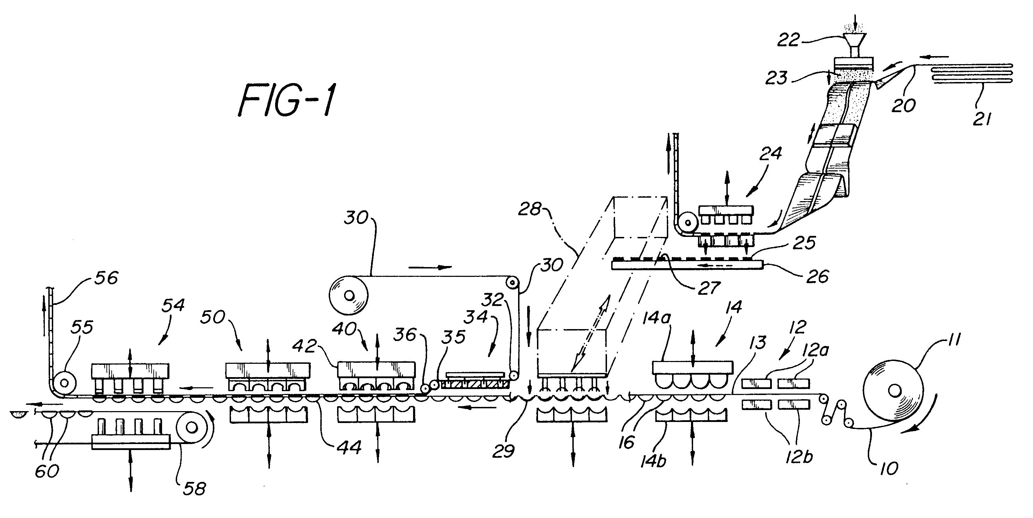

This invention will now be described in more detail with reference to the drawings. As generally depicted schematically in Fig. 1, an ethylene-containing foam sheet material 10 is provided in roll form at 11. The foam material is preferably a flexible polyethylene-containing closed-cell foam sheet having thickness of 2.54 to 6.35 mm (0.10 to 0.25 inch). The sheet is passed intermittently through a heating step 12 where it is rapidly radiantly heated on its upper and lower sides to 54.4 to 121.1°C (130 to 250° F) temperature to soften the foam material. The heated sheet at 13 is then passed to an adjacent die forming step 14, where the heated foam sheet is drawn to form multiple shells 16 which are integrally attached to the sheet 10. The shells have a depth at least about 10 times the foam sheet original thickness and preferably 12 to 20 times the sheet thickness. The foam sheet is moved forward intermittently by a distance equal to the total width of the shells being simultaneously die formed in the foam sheet.

A fibrous web material is provided at 20, usually in a folded form provided at 21 or a similar convenient form. The web sheet is composed of dual layers of non-woven fibers such a polyester, the layers being corrugated transversely to the length of the sheet 20. The web sheet is preferably filled at 22 with an absorbent powder 23 to a powder/web weight ratio of between about 1/1 and 5/1. The resulting powder-filled web material is then die cut at 24 to produce a plurality of individual flexible web pieces 25. These web pieces 25 are conveyed on a shuttle conveyor 26 to a position 27, from which the pieces 25 are each picked up by a carrier device 28 oriented above the formed shells 16 and each conveyed and inserted into a formed shell 16. While the web pieces 25 are being conveyed to the shells 16, they are simultaneously crimped longitudinally so as to be inserted snugly into the formed foam shells.

The formed shells 16 each containing a crimped fibrous web piece 29 are next covered with a porous facing sheet provided at 30. This facing sheet 30 is first guided by roller 32 to a sheet slitting step 34, where the sheet 30 is selectively transversely slit at spaced intervals located intermediate the shells 16 to permit the facing sheet to move laterally as needed to conform to the upper surface of the formed shells 16. Then the slit facing sheet at 35 is guided by rollers 36 downwardly to contact the foam sheet 10 ahead of sealing step at 40, which preferably uses a heat sealing die unit. This die unit rapidly heats the slit facing sheet 35 and upper surface of the plastic foam shells 16 to their softening temperature usually 54.4 to 121.1°C (130-250° F) by heating step 42, and rapidly heat seals the facing sheet onto the perimeter of shell 16.

The resulting formed shells with heat sealed cover sheet at 44 are next passed to die cutting step 50, where the formed and covered shells 29 are die cut from the sheet around the shell perimeter at all points except for a link located at each end of each shell. The resulting partially severed shells 16 are then passed on to a punch out step 54, where the partially severed shells are punched out from the surrounding sheet 10. The resulting scrap sheet at 56 from which the shells have been removed is withdrawn over roller 55. The resulting complete products 60 are guided downward onto a conveyor belt 58, which carries the products on to inspection and packaging steps (not shown).

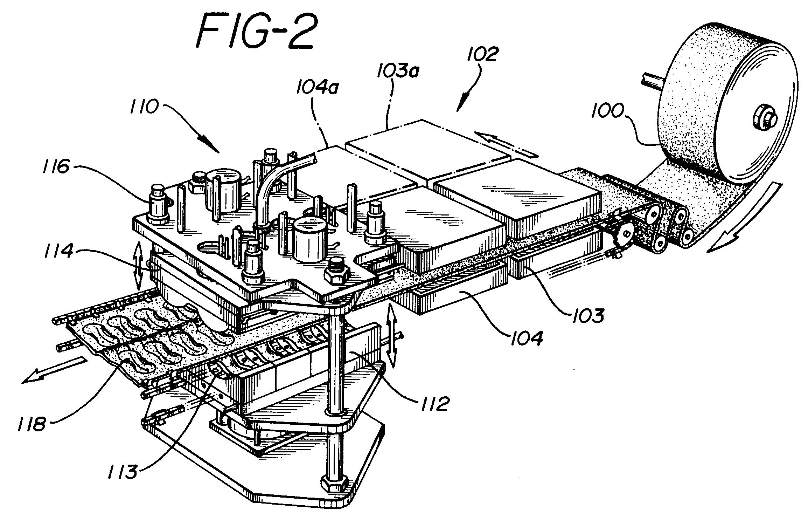

The foam sheet heating and die forming step of the invention will now be described in greater detail with reference to Fig. 2. The foam sheet from roll 100 is rapidly radiantly heated at 102 by two radiant heaters 103 and 104 arranged in series, with the first heater 103 heating the foam from ambient temperature to 65.5 to 93.3°C (150 to 200°F) and the second heater 104 heating the foam to a final temperature of 93.3 to 121.1°C (200-250°F). The heaters can each be moved aside transversely to positions 103a and 104a when regular movement of the foam sheet is interrupted for any reason, to avoid excessive heating and damage to the foam.

The heated foam sheet is then passed intermittently to forming die assembly 110, where it is die formed by lower die unit 112 containing cavities 113 and upper die unit 114 containing plugs. The die units 112 and 114 are closely matched dimensionally and are guided by vertical guide rods 116. Formed shells 118 are produced integrally attached to the foam sheet 100.

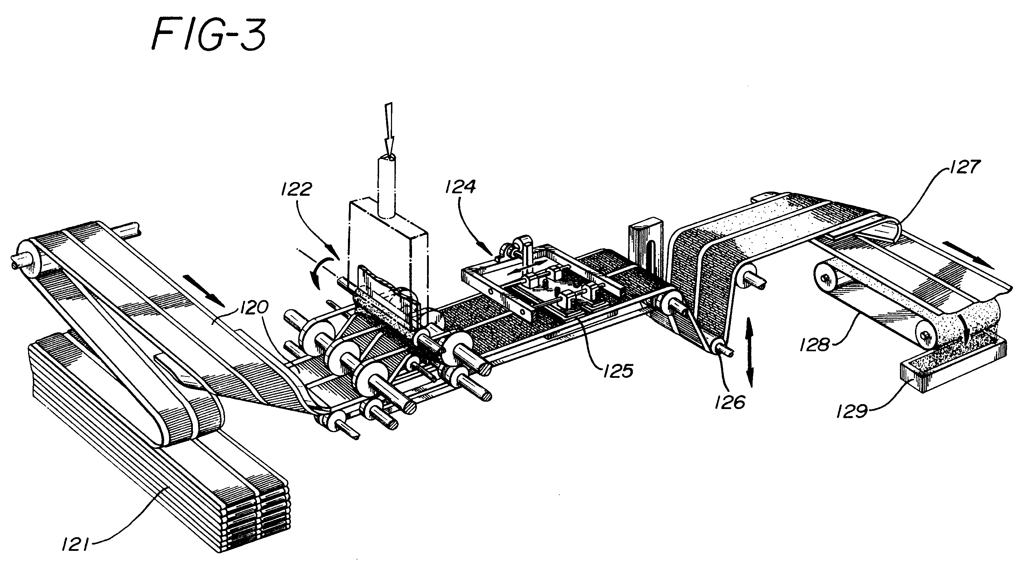

The fibrous web powder filling step is additionally shown by Fig. 3. A fibrous web material 120, is provided in folded form at 121 and continuously fed to powder addition step at 122. The powder is preferably rubbed into the web at 124 using a reciprocating pad 122. The web continuous forward motion is converted to an intermittent motion while being passed below a vertically movable roller at 126. The web is inverted at roll bend 127 so as to place the powder on the lower side of the web. Any surplus powder which falls from the web is collected by belt 128 and receptacle 129.

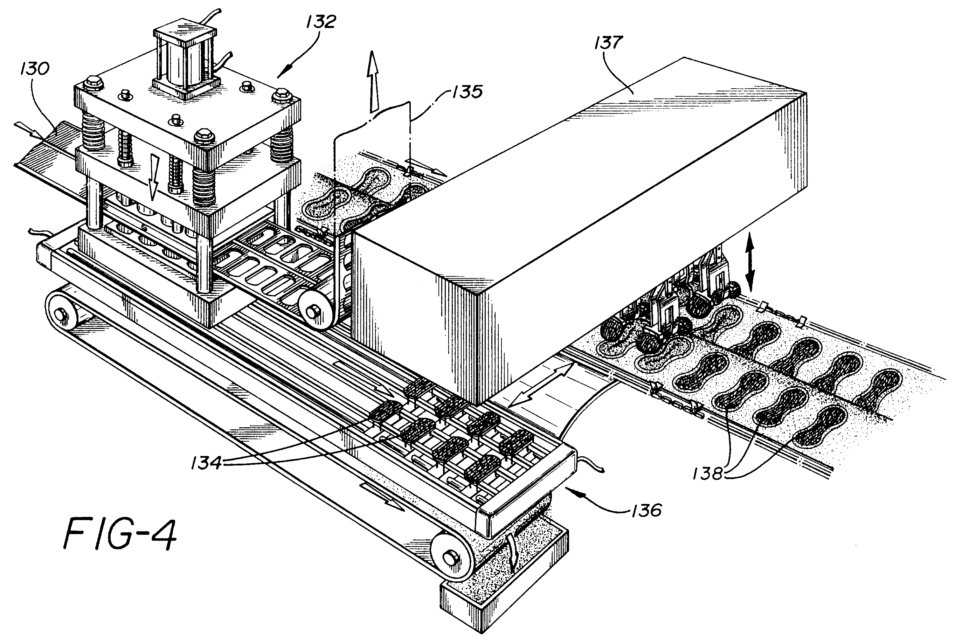

As additionally shown by Fig. 4, the powder-filled web at 130 is then die cut by cutting die assembly 132 to produce individual generally flat web pieces 134 with the remaining scrap strip being withdrawn overhead at 135. The web pieces 134 are received and moved forward on conveyor 136 and are then picked up and crimped longitudinally and transferred laterally by carrier 137 and the crimped web pieces are placed into the formed shells 138.

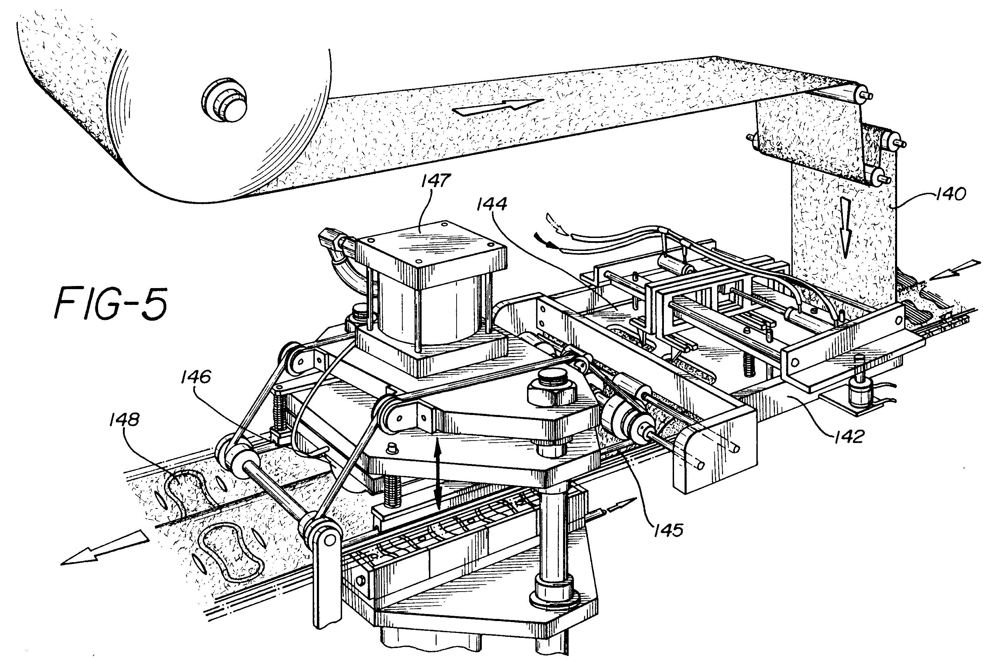

Following placing the web pieces into formed shells 138, the facing sheet is attached to the formed shells, as additionally shown in perspective by Fig. 5. The fibrous facing sheet provided at 140 is clamped at 142 and transversely selectively slit at 144 at locations between adjacent shells to facilitate selective lateral movement off the facing sheet towards the shells during the subsequent heat sealing step. The slit sheet 145 is then guided downwardly to be adjacent the foam shells 138, after which heated plugs 146 are moved downwardly by actuator piston 147 to smoothly heat seal the facing sheet 140 onto the perimeter of the formed shells, to produce covered shells at 148.

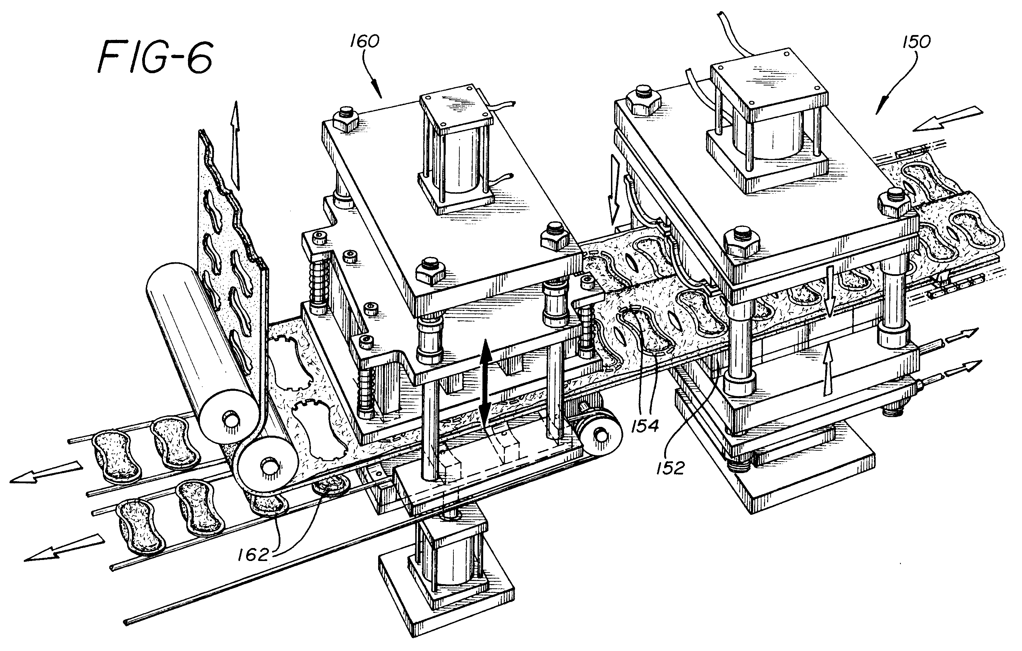

The covered shells 148 are next passed to die cutting step 150, as is shown in greater detail by perspective view Fig. 6. The covered shells are received into a shaped cavity off lower die unit 152, and are then clamped and die cut at their perimeter by vertically movable serrated knives. The shells are severed from the surrounding foam sheet, except at least one link 154 remaining at each end of the product. The die cut products are then passed to punch out step at 160 where they are punched out to provide the completed formed shell product at 162.

This invention will be further described by reference to the following examples of producing a specific formed product, which examples should not be construed as limiting the scope of the invention.

### Example 1

A closed cell polyethylene foam elongated sheet 3.175 mm (0.125 inch) thick and 40.64 cm (16 inch) wide is gripped along its edges by a conveyor chain and passed between dual radiant heating units located above and below the sheet with each heating unit containing an electrically heated ceramic plate. Performance characteristics for the heating step are as follows:

| | |
|---|---|
| Heater spacing from foam sheet | 3.81 cm (1.5 in) |
| Heater temperature, | 260°C (500°F) |
| Foam sheet heated temperature, | 65.5°C (150°F) |
| Foam sheet heating rate, | 16.7°C/sec (30°F/sec). |

The heated foam sheet is passed forward to an adjacent die forming step, including a lower die unit provided with dual cavities and an upper die unit having dual plugs which each interfit with the lower die cavities. Both die units are cooled by a coolant circulating through flow passages provided therein. A central portion of the foam sheet is first withdrawn partly into each die cavity by vacuum pressure, after which the upper die unit descends and closes together to clamp the foam therebetween. Then the plug descends and deforms the heated flexible foam downwardly into the die cavity while cooling the foam sheet by contact with both the die parts. Substantially simultaneously with the plug descent, pressurized air is injected into a clearance space between the plug and the deformed foam sheet to force the foam sheet laterally into side grooves of the die cavity to form a shell. Then after a brief 0.5 sec pause to permit further cooling and dimensional stabilization of the formed foam shell, the die units are separated and the formed sheet containing the formed shell is moved intermittently forward and the forming step repeated.
Important performance characteristics for the die forming step are as follows:

| | |
|---|---|
| Foam sheet temperature, | 65.6°C (150°F) |
| Die cavity wall temperature, | 10°C (50°F) |
| Die plug temperature, | 21.1°C (70°F) |
| Foam shell cooling rate, °F/sec | 27.8°C/sec (50°F/sec) |
| Ratio maximum shell depth to foam sheet original thickness | 15 |
| Ratio shell min. thickness to foam sheet thickness | 0.4 |

Each formed shell produced has dimensions of 10.16 cm (4 inch) wide by 19.05 cm (7.5 inches) long and 2.54 cm (1 inch) deep.

### Example 2

A continuous elongated corrugated fibrous web is composed of two superimposed layers of fibrous material having a flat embossed portion extending along each side and in a middle portion of the web. The corrugated web is gripped on both sides of each embossed portion by a set of mating belts and is drawn over a bend roller to open the corrugations on the upper side, while an asorbent powder is deposited by gravity feed into the opened spaces between the adjacent corrugations. The ratio of the bending roller radius to web thickness is 1.0. The fibrous web and powder filling step have characteristics as listed in Table 2 below.

**Table 2**

| Characteristics of Corrugated Fibrous Web and Powder | |
|---|---|
| Web material | polyester fibers |
| Web thickness, | 1.397-2.54 cm (0.55-1.0 inches) |
| Web width, inches | 45.72 to 50.8 cm (18-20 inches) |
| Number of web corrugations | 1.38-1.57 per cm (3.5-4 per inch) |
| Powder composition | potassium acrylate |
| Powder particles size range | 100-400 µm |
| Absorbent powder added to web weight % of web | 100-150 |

After the powder is deposited in the web upper layer, the web is passed under a rubbing pad oscillating at 30 strokes per minute which exerts a light pressure of 96-144 Pa (2-3 pounds/ft²) on the powder to force it further into the web corrugations, so as to provide a desired powder/web weight ratio of about 1.0/1 for the fibrous web structure.

### Example 3

A continuous fibrous web from Example 2 composed of two superimposed corrugated layers of fibrous material and having a flat embossed portion extending along each side and in a middle portion of the web, contains an absorbent powder provided into spaces between the corrugations as in Example 2. The web is passed to a cutting die including a lower body unit containing eight openings each having the shape of the web pieces and an upper body unit containing eight punches each closely interfitting into the openings of the lower die unit. The web is fed intermittently to the cutting die assembly which die cuts out the individual web pieces and forces them downwardly through the openings onto upwardly extending pins of a shuttle conveyor.The resulting web pieces have characteristics as listed in Table 3 below.

**Table 3**

| Characteristics of Corrugated Fibrous Web Pieces | |
|---|---|
| Web material | polyester fibers |
| Web thickness, inches | 1.27 to 2.54 cm (0.50-1.0 inches) |
| Web width, inches | 45.72 to 50.8 cm (18-20 inches) |
| Number of corrugations | 1.38 to 1.57 per cm (3.5-4 per inch) |
| Absorbent powder contained in web, weight % of web | 100-150 |
| Powder particle size range | 100-400 µm |
| Number of web pieces | 8 |

The eight web pieces are each 15.24 cm (6 inches) long by 7.62 cm (3 inches) wide and 1.27 cm (0.5 inch) thick, and are arranged in two rows of four pieces each on the shuttle conveyor. The adjacent pieces in each row are moved apart from each other by 2.54 cm (1 inch) movement of the segments off the shuttle conveyor, each segment containing two upwardly extending pins which penetrate and move the web pieces apart.

### Example 4

Eight flat fibrous web pieces are provided from a die cutting operation onto support rails of a shuttle conveyor device as in Example 3. The web pieces are each longitudinally corrugated and composed of dual layers of polyester fibers, and each have dimensions of 15.24 cm (6 inches) long by 7.62 cm (3 inches) wide and 1.27 cm (0.5 inches) thick. The web pieces are oriented in a 2 x 4 pattern, i.e. with two rows off four pieces each in an end-to-end relation with each other as generally shown in Fig. 4. The web pieces are placed onto dual upwardly extending pins attached to blocks of a shuttle conveyor, and the pieces are moved apart laterally from each other by 3.81 cm (1.5 inches).

The eight web pieces are each grasped simultaneously and are longitudinally crimped by dual clamp fingers, and the pieces are picked up and transferred laterally to above a plurality of formed shells in a foam sheet arranged in a similar 2 x 4 pattern as in Example 1. During the lateral transfer movement, the end-to-end spacing between the web pieces is reduced by 2.54 cm (1 inch), i.e. the web pieces are moved closer together. The respaced web pieces are then each inserted into a corresponding concave shaped formed shell of the foam sheet. The cycle time for the entire picking and placing maneuver is 3 seconds.

### Example 5

A flexible polyethylene foam sheet is provided in which elongated shaped shells have been formed and web pieces are inserted as in Example 4. Each shell is 16.51 cm (6.5 inches) long and 7.62 cm (3 inches) wide by 1.90 cm (0.75 inch) deep by and has a central portion which is depressed by 2.54 cm (1 inch) relative to the shell end portions. A thin facing sheet composed of a polyester non-woven liquid permeable web is provided above the foam sheet. Other characteristics of the foam and facing sheets are as follows:

| | |
|---|---|
| Foam sheet material | closed-cell polyethylene |
| Foam sheet thickness, | 3.17 mm (0.125 in.) |
| Foam sheet width, | 40.64 cm (16 in) |
| Facing sheet material | non-woven polyester fibrous web |
| Facing sheet thickness, | 0.254 mm (0.010 in) |
| Facing sheet width, | 48.26 cm (19 in) |

The facing sheet is first clamped between mating plates and then slit transversely within its central portion at spaced intervals located between the formed shells in the foam sheet.

The facing sheet is moved adjacent the foam sheet and both sheets are indexed forward to a heat sealing die assembly. The formed shells, which each contain a corrugated fibrous insert piece, are each seated into a lower cavity off the die assembly lower unit with the aid of vacuum pressure applied to holes provided in each the cavity. Then the upper die unit containing an outer clamp and heated plugs descends so that the facing sheet is clamped onto the foam sheet and the plugs interfit within the die cavities. During the clamping the central portions of the facing sheet move laterally toward the shells, then the heated plugs rapidly soften the fibrous facing sheet and the foam sheet upper surface and heat seals the facing sheet onto the foam sheet at location around the perimeter off the formed shells. Important characteristics of the heat sealing step are as follows:

| | |
|---|---|
| Facing sheet slit length, | 12.7 cm (5 in) |
| Foam sheet softening temp. | 93.3°C (200°F) |
| Sealing plug temperature, | 93.3°C (300°F) |
| Facing sheet sealing time, | 2 sec |

Following the heat sealing step for the facing sheet, the mating die units are separated and the foam sheet containing the covered shells is indexed forward to a subsequent shell cut out step.

### Example 6

An ethylene-containing foam sheet contains multiple formed shell products aligned in two parallel rows, as described in Example 5. The foam sheet is 5.08 mm (0.20 inches) thick and 40.64 cm (16 inches) wide, and the shells are each 8.64 cm (3.4 inches) wide, and 19.05 cm (7.5 inches) long, with the shells being covered by a fibrous polyester facing sheet heat bonded onto the foam sheet as in Example 5. The foam sheet integrally containing the formed shells is fed to a cutting die assembly containing eight matched cutting die units. The cutting dies are closed to first clamp the foam sheet and shell product, and then to sever the shell from the foam sheet after which the sheet is intermittently indexed forward by a distance equal to the width of four shells.

Important characteristics of the die cutting step are as follows:

| | |
|---|---|
| Cutting blade width, | 0.508 mm (0.020 in) |
| Blade teeth spacing, | 2.54 mm (0.10 in) |
| Cutting blade material | spring steel |
| Die groove width, | 1.27 mm (0.050 in) |

Following cut out of the foam shell products, the foam sheet is indexed forward to a punch out step in which the individual products are severed from the foam sheet and are guided downwardly onto a moving belt which continuously removes the products.

The step of forming the shells from the polyethylene-containing foam is described in more detail in our copending European application EP-A-0 256 868, claiming priority from USSN 897 168 of 15th August, 1986 (JBD 111).

The step of providing powder into the corrugated web is described in more detail in our copending European application EP-A-0 256 869, claiming priority from USSN 897 171 of 15th August, 1986 (JBD 112).

The step of picking and placing the die-cut webs into the foam shells is described in more detail in our copending European application EP-A-0 256 870 claiming priority from USSN 897 224 of 15th August, 1986 (JBD 114).

Although this invention has been described broadly and also in terms of a preferred embodiment, it will be understood that modifications and variations in the materials and method steps can be made within the scope of the invention, which is defined by the following claims.

## Claims

1. A process for making a formed flexible plastic foam product (60) containing a corrugated fibrous web insert piece, comprising:
(a) providing a flexible foam sheet material (10) and rapidly heating the foam material to 130-250°F (54-121°C) temperature;
(b) die forming the heated foam sheet to provide at least one formed shell (16) integrally attached to the foam sheet (10), said shell (16) having a depth at least about 10 times the sheet original thickness, said die forming step (14) being performed by effecting relative movement between cooperating first and second die units, said first die unit (112) having a cavity (113) shaped to the configuration of said shell (16) and said second die unit (114) having a shaped plug adapted to mate within said cavity (113) and to draw a portion of said heated foam sheet into said cavity in response to said relative movement;
(c) providing corrugated fibrous web material (20) and die cutting at least one web piece (27) from the web;
(d) placing each said cut out web piece (27) into each said formed shell (16);
(e) applying a thin porous facing sheet (30) over each said web piece (27) in the formed shell (16), selectively transversely slitting the porous facing sheet (30), and thereafter heat sealing the facing sheet (30) onto the perimeter of each said formed shell (16); and
(f) cutting the formed shell (16) and attached facing sheet (30) at the perimeter of the shell, and thereafter removing the cut out shells from the foam sheet to provide a plurality of the formed plastic foam products (60).

2. The process of claim 1, wherein the flexible foam sheet material (1) is an ethylene-containing material.

3. The process of claim 1 or claim 2, wherein the foam sheet material (10) is heated in step (a) by radiant heating (12).

4. The process of any one of claims 1 to 3, wherein the shell (16) has a depth equal to 12 to 20 times the foam sheet original thickness.

5. The process of claim 4, including additionally depositing a powder material (23) within the corrugations provided in the fibrous web (20) before die cutting the web.

6. The process of claim 5, wherein the corrugated fibrous web (20) containing the powder (23) is inverted to place the powder on the lower side of the web prior to die cutting out the web piece from the web.

7. The process of any one of claims 1 to 6, wherein the web piece (27) is crimped longitudinally during placing of the piece in the formed shell (16).

8. The process of any one of claims 1 to 7, wherein the facing sheet (30) is fibrous.

9. The process of any one of claims 1 to 8, wherein the facing sheet (30) is heated to 54.4 to 121.1°C (130-250°F) for sealing the facing sheet (30) onto the formed shell (16).

10. The process of any one of claims 1 to 9, wherein the facing sheet (3) is heat sealed onto a non-linear upper surface of the formed shell (16).

11. The process of any one of claims 1 to 10, wherein the faced formed shell (16) is die cut in step (f).

12. The process of any one of claims 1 to 11, including applying a sealing tape to the convex side of the formed shell (16) before cutting the shell from the foam sheet.

13. The process of any one of claims 1 to 12, wherein multiple formed shells (16) are produced during step (b), multiple web pieces (27) are produced during step (c) and multiple formed plastic foam products (60) are produced in step (f).

14. The process of claim 13, wherein the foam sheet (10) is moved intermittently through the heating area.

15. The process of claim 13 or claim 14, wherein the product (60) is formed at a rate of 10 to 30 cycles per minute.

## Patentansprüche

1. Verfahren zum Herstellen eines geformten, flexiblen Kunststoffschaumerzeugnisses (60), enthaltend ein gewelltes Faservlies-Einsatzteil, bestehend aus:
(a) dem Bereitstellen eines flexiblen Schaumstoff-Schichtmaterials (10) und schnelles Erhitzen des Schaumstoffmaterials auf eine Temperatur von 130 - 250 °F (54 - 121 °C);
(b) dem Gesenkformen der erhitzten Schaumstoffschicht zur Bildung mindestens einer geformten Schale (16), die an der Schaumstoffschicht (10) integriert befestigt ist, wobei die Schale (16) eine Tiefe von mindestens etwa dem 10-fachen der ursprünglichen Dicke der Schicht hat, und die genannte Gesenkformstufe (14) durch Ausführung einer relativen Bewegung zwischen zusammenwirkenden ersten und zweiten Gesenkeinheiten durchgeführt wird, wobei die genannte erste Gesenkeinheit (112) einen Matrizenhohlraum (113) hat, der an die Form der Schale (16) angepaßt ist und die zweite Gesenkeinrichtung (114) einen Formstempel hat, der, in den Matrizenhohlraum (113) hineinpaßt und einen Teil der erhitzten Schaumstoffschicht in den Matrizenhohlraum in Abhängigkeit von der genannten Relativbewegung hineinzieht;
(c) dem Bereitstellen gewellten Faservliesmaterials (20) und Ausstanzen mindestens eines Vliesteils (27) aus dem Vlies;
(d) dem Einlegen jedes ausgeschnittenen Vliesteils (27) in jede geformte Schale (16);
(e) dem Aufbringen einer dünnen, porösen Außenschicht (30) auf jedes Vliesteil (27) in der geformten Schale (16), dem wahlweisen Querschneiden der porösen Außenschicht (30) und anschließendes Heißsiegeln der Außenschicht (30) am Umfang jeder geformten Schale (16); und
(f) Ausschneiden der geformten Schale (16) und der daran befestigten Außenschicht (30) an dem Umfang der Schale und anschließendes Entfernen der ausgeschnittenen Schalen von der Schaumstoffschicht, um zum Zurverfügungstellen eine Mehrzahl der geformten Kunststoff-Schaumerzeugnisse (60) zur Verfügung zu stellen.

2. Verfahren nach Anspruch 1, bei dem das flexible Schaumstoff-Schichtmaterial (10) ein ethylenhaltiges Material ist.

3. Verfahren nach Anspruch 1 oder Anspruch 2, wobei das Schaumstoff-Schichtmaterial (10) in Stufe (a) durch Strahlungsheizung (12) erhitzt wird.

4. Verfahren nach einem der Ansprüche 1 bis 3, wobei die Schale (16) eine Tiefe des 12- bis 20-fachen der ursprünglichen Dicke der Schaumstoffschicht hat.

5. Verfahren nach Anspruch 4, umfassend ein zusätzliches Einbringen eines pulverförmigen Materials (23) innerhalb der in dem Faservlies (20) vorgesehenen Wellen vor dem Ausstanzen des Vlieses.

6. Verfahren nach Anspruch 5, bei dem das gewellte, das Pulver (23) enthaltende Faservlies (20) umgedreht wird, um das Pulver auf der Unterseite des Vlieses vor dem Ausstanzen des Vliesteils aus dem Vlies zu plazieren.

7. Verfahren nach einem der Ansprüche 1 bis 6, bei dem das Vliesteil (27) in Längsrichtung während des Plazierens des Teiles in der geformten Schale (16) gekrimpt wird.

8. Verfahren nach einem der Ansprüche 1 bis 7, wobei die Außenschicht (30) faserig ist.

9. Verfahren nach einem der Ansprüche 1 bis 8, wobei die Außenschicht (30) auf 54,4 bis 121,1 °C (130 - 250 °F) zum Siegeln der Außenschicht (30) auf der geformten Schale (16) erhitzt wird.

10. Verfahren nach einem der Ansprüche 1 bis 9, wobei die Außenschicht (30) auf eine nicht-lineare Oberseite der geformten Schale (16) heißgesiegelt wird.

11. Verfahren nach einem der Ansprüche 1 bis 10, wobei die kaschierte, geformte Schale (16) in Stufe (f) ausgestanzt wird.

12. Verfahren nach einem der Ansprüche 1 bis 11, umfassend das Aufbringen eines Dichtungsstreifens auf die konvexe Seite der geformten Schale (16) vor dem Ausschneiden der Schale aus der Schaumstoffschicht.

13. Verfahren nach einem der Ansprüche 1 bis 12, wobei mehrere geformte Schalen (16) während der Stufe (b) erzeugt werden, mehrere Vliesteile (27) während der Stufe (c) erzeugt werden, und mehrere geformte Kunststoffschaumerzeugnisse (60) in Stufe (f) erzeugt werden.

14. Verfahren nach Anspruch 13, wobei die Schaumstoffschicht (10) intermittierend durch die Erhitzungszone bewegt wird.

15. Verfahren nach Anspruch 13 oder Anspruch 14, wobei das Erzeugnis (60) mit einer Geschwindigkeit von 10 bis 30 Arbeitszyklen pro Minute hergestellt wird.

## Revendications

1. Procédé de fabrication de produits façonnés flexibles plastiques en mousse (60) contenant un coupon de tissu inséré en fibre striée, comprenant les étapes de:
(a) fourniture d'une feuille flexible de matière en mousse (10) et chauffage rapide de la matière en mousse à une température comprise entre 54 et 121°C (130 et 250°F);
(b) estampage de la feuille en mousse chauffée pour fournir au moins une cellule façonnée (16) attachée intégralement à la feuille en mousse (10), ladite cellule (16) ayant une profondeur d'environ 10 fois l'épaisseur initiale de la feuille, ladite étape d'estampage (14) étant réalisée en effectuant un mouvement relatif entre des premières et secondes unités d'estampage coopérant entre elles, ladite première unité d'estampage (112) ayant une cavité (113) de la forme de la configuration de ladite cellule (16) et ladite seconde unité d'estampage (114) ayant une forme de moule adaptée pour s'accoupler à ladite cavité (113) et pour étirer une partie de ladite feuille en mousse chauffée dans ladite cavité en réponse audit mouvement relatif;
(c) fourniture d'une matière en tissu en fibre striée (20) et découpe à l'emporte-pièce d'au moins un coupon (27) de tissu;
(d) pose de chacun desdits coupons de tissu découpés (27) dans chacune desdites cellules façonnées (16);
(e) application d'une mince feuille de protection perméable (30) sur chacun desdits coupons de tissu (27) dans la cellule façonnée (16), incision d'une fente transversalement et sélectivement de la feuille mince de protection perméable (30), et ensuite thermosoudure de la feuille de protection (30) sur le périmètre de chacune desdites cellules façonnées (16); et
(f) découpe de la cellule façonnée (16) et de la feuille de protection fixée (30) sur le périmètre de la cellule, et ensuite séparation des cellules découpées de la feuille en mousse pour fournir une pluralité de produits plastiques en mousse façonnés (60).

2. Procédé selon la revendication 1, dans lequel la feuille flexible de matière en mousse (1) est une matière contenant de l'éthylène.

3. Procédé selon la revendication 1 ou la revendication 2, dans lequel la feuille de matière en mousse (10) est chauffée à l'étape (a) par un élément chauffant par rayonnement (12).

4. Procédé selon l'une quelconque des revendications 1 à 2, dans lequel la cellule (16) a une profondeur égale de 12 à 20 fois l'épaisseur de la feuille en mousse initiale.

5. Procédé selon la revendication 4 comprenant de plus la déposition d'une matière en poudre (23) dans les stries façonnées du tissu en fibre (20) avant découpe à l'emporte-pièce de la bande.

6. Procédé selon la revendication 5, dans lequel la bande en fibre striée (20) contenant la poudre (23) est retournée pour placer la poudre sur le côté inférieur de la bande avant la découpe à l'emporte-pièce du coupon de tissu dans la bande.

7. Procédé selon l'une quelconque des revendications 1 à 6, dans lequel le coupon de tissu (27) est plissé longitudinalement pendant la pose du coupon dans la cellule façonnée (16).

8. Procédé selon l'une quelconque des revendications 1 à 7, dans lequel la feuille de protection (30) est en fibre.

9. Procédé selon l'une quelconque des revendications 1 à 8, dans lequel la feuille de protection (30) est chauffée à une température comprise entre 54,4 et 121,1°C (130 et 250°F) pour souder la feuille de protection (30) sur la cellule façonnée (16).

10. Procédé selon l'une quelconque des revendications 1 à 9, dans laquelle la feuille de protection (3) est thermosoudée sur une surface supérieure non-linéaire de la cellule façonnée (16).

11. Procédé selon l'une quelconque des revendications 1 à 10, dans lequel la cellule façonnée (16) est découpée à l'emporte-pièce à l'étape (f).

12. Procédé selon l'une quelconque des revendications 1 à 11, comprenant l'application d'une bande de fixation sur le côté convexe de la cellule façonnée (16) avant de découper la cellule de la feuille en mousse.

13. Procédé selon l'une quelconque des revendications 1 à 12, dans lequel de multiples cellules façonnées (16) sont fabriquées à l'étape (b), de multiples coupons de tissu (27) sont fabriqués à l'étape (c) et de multiples produits façonnés en mousse plastique (60) sont fabriqués à l'étape (f).

14. Procédé selon la revendication 13, dans lequel la feuille on mousse (10) est déplacée par intermittence à travers la zone de chauffage.

15. Procédé selon la revendication 13 ou la revendication 14, dans lequel le produit (60) est fabriqué à une vitesse de 10 à 30 cycles par minute.
